Europäisches Patentamt

European Patent Office

Office européen des brevets

(11) Publication number: 0 211 079
A1

## (12) EUROPEAN PATENT APPLICATION
published in accordance with Art. 158(3) EPC

(21) Application number: 85900763.5

(22) Date of filing: 31.01.85

Data of the international application taken as a basis:

(86) International application number:
PCT/JP85/00041

(87) International publication number:
WO86/04501 (14.08.86 86/18)

(51) Int. Cl.⁴: A 61 K 7/00
A 61 K 9/48, A 61 J 3/07
A 23 P 1/04

(43) Date of publication of application:
25.02.87 Bulletin 87/9

(84) Designated Contracting States:
AT BE CH DE FR GB LI LU NL SE

(71) Applicant: Fujisawa Pharmaceutical Co., Ltd.
3, Doshomachi 4-chome Higashi-ku
Osaka-shi, Osaka 541(JP)

(72) Inventor: UEDA, Yoshio
No. 3-5-204, Mikagenakamachi 1-chome
Higashinada-ku, Kobe-shi Hyogo 658(JP)

(72) Inventor: SHIMOJO, Fumio
No. 2-13, Daiwahigashi 2-chome
Kawanishi-shi Hyogo 666-01(JP)

(72) Inventor: SAITO, Takashi
No. 2-10, Midorigaoka 2-chome
Ikeda-shi Osaka 563(JP)

(74) Representative: Tiedtke, Harro, Dipl.-Ing. et al,
Patentanwaltsbüro Tiedtke-Bühling-Kinne-
Grupe-Pellmann-Grams-Struif Bavariaring 4 Postfach 20
24 03
D-8000 München 2(DE)

(54) SOFT MULTI-CHAMBER CAPSULE AND METHOD OF AND APPARATUS FOR MANUFACTURING SAME.

(57) A soft multi-chamber capsule consists of a covering, the inner space of which is divided into a plurality of chambers by at least one partition. These chambers contain materials, for example, medicine, cosmetics or food. The number of such chambers is usually two, and the space between first and second coverings is divided into two chambers by a partition provided therebetween. Such a soft multi-chamber capsule is manufactured by feeding a first film, which is to form the first covering, a second film, which is to form the second covering, and a third film, which is to form the partition between a pair of die rolls in a capsule molding apparatus continuously so that the third film is positioned between the other two films, while introducing materials into the space between the capsule-defining portions of the third and first films and the space between the capsule-defining portions of the third and first films; and joining under pressure these three films except their respective capsule-defining portions.

FIG.1

**0211079**

S P E C I F I C A T I O N

MULTICELLULAR SOFT CAPSULES AND PROCESS

AND APPARATUS FOR PRODUCING SAME

TECHNICAL FIELD

The present invention relates to multicellular soft capsules containing a medicinal, cosmetics or food, and to a process and an apparatus for producing the same.

BACKGROUND ART

Soft capsules having, for example, a medicinal enclosed therein are known which comprise a first shell at one side and a second shell at the other side and which has only one space between the shells for containing the contents. Such capsules are produced by the planar process which comprises placing over a lower die a first film for forming the first shell, pouring a liquid medicinal onto the film, placing over the medicinal a second film for forming the second shell, placing an upper die over the second film and applying pressure to the upper die to compress the two films together except at capsule forming portions thereof so that the medicinal is enclosed in a space between the two films at the capsule forming portions, or by the rotary process which comprises continuously feeding first and second films to the space between a pair of die rolls and compressing the two films together except at capsule forming portions thereof while placing a medicinal into a space between the two films

0211079

at the capsule forming portions. However, the conventional processes are unable to form more than one containing space between the first shell and the second shell, so that it is impossible to stably enclose in a single capsule two or more medications which, for example, are pharmaceutically incompatible. Further when there is a need for a drug which is decomposed in the stomach and a drug which is not so, it is necessary to separately administer a capsule made of an enteric film and another capsule made of an intragastrically soluble film. Also when there is a need for a medicinal with sustained release action, it is necessary to separately give a rapid release capsule of rapidly soluble film and a delayed release capsule of prolonged release or enteric film.

An object of the present invention is to provide a soft capsule of novel structure which, although single, is adapted to stably enclose at least two kinds of incompatible contents and which, although in the form of a single soft capsule, can be made to have one portion of rapidly soluble or intragastrically soluble properties and the other portion of prolonged release or enteric properties, or to have one portion with a rapid release action and the other portion with a delayed release action.

Another object of the present invention is to provide a process and an apparatus for producing a soft capsule of novel structure in which the interior space defined by a shell is divided into a plurality of compartments by at least one partition.

## DISCLOSURE OF THE INVENTION

The multicellular soft capsule of the present invention has a shell defining an interior space which is divided into a plurality of compartments or cells by at least one partition.

With the multicellular soft capsule of the present invention, different contents can be enclosed in the different cells. One portion of the capsule can be made different from the other portion thereof in properties by suitably selectively determining the properties of the partition and of the constituent portions of the shell as well as the properties of contents to be contained in the plurality of cells. Accordingly, two or more kinds of medicinals which, for example, are incompatible with each other can be stably enclosed in a single capsule and can be administered in the form of only one capsule. A single sustained release capsule can be obtained by enclosing a rapid release medicinal and a prolonged release medicinal separately in the plurality of cells. The partition and a shell portion on one side thereof may be made to have prolonged release or enteric properties, and the other shell portion made rapidly soluble, to render one portion of the capsule slow acting and the other portion thereof rapidly acting, whereby a single sustained release capsule can also be obtained. Furthermore when the partition and a shell portion on one side thereof are made enteric with the remaining portion made soluble in the stomach, a medicinal which is decomposed in the stomach and another medicinal which is not so can be administered as enclosed in a single capsule.

The process of the invention produces multicellular soft capsules comprising a first shell and a second shell defining a space therebetween and at least one partition provided between the shells and dividing the space into a plurality of cells, the process comprising feeding to capsule forming means a first film for forming the first shell, a second film for forming the second shell and at least one sheet of third film for forming the partition with the third film positioned between the first and second films, and compressing the first, second and third films together except at capsule forming portions thereof so that contents are placed into spaces between the films at the capsule forming portions.

The apparatus of the invention produces multicellular soft capsules comprising a first shell and a second shell defining a space therebetween and at least one partition provided between the shells and dividing the space into a plurality of cells, the apparatus comprising capsule forming

-4-

means composed of a pair of die rolls, first film feeding means for continuously feeding between the die rolls a first film for forming the first shell, second film feeding means for continuously feeding between the die rolls a second film for forming the second shell, third film feeding means for continuously feeding at least one sheet of third film for forming the partition from between the first film and the second film to between the die rolls, and a plurality of contents feeding means for separately placing contents into spaces between the films at capsule forming portions thereof.

The process and apparatus of the invention produce multicellular soft capsules having an interior space which is defined by a shell and divided into a plurality of cells by a partition.

BRIEF DESCRIPTION OF THE DRAWINGS

Fig. 1 is a view in vertical section showing an example of multicellular soft capsule according to the invention;

Fig. 2 is a fragmentary view in vertical section showing an apparatus of the invention for producing multicellular soft capsules;

Fig. 3 is a view in vertical section showing a modified example of multicellular soft capsule; and

Fig. 4 is a view in vertical section showing another modified example of multicellular soft capsule.

DESCRIPTION OF THE PREFERRED EMBODIMENTS

The present invention will be described below in greater detail with reference to the accompanying drawings.

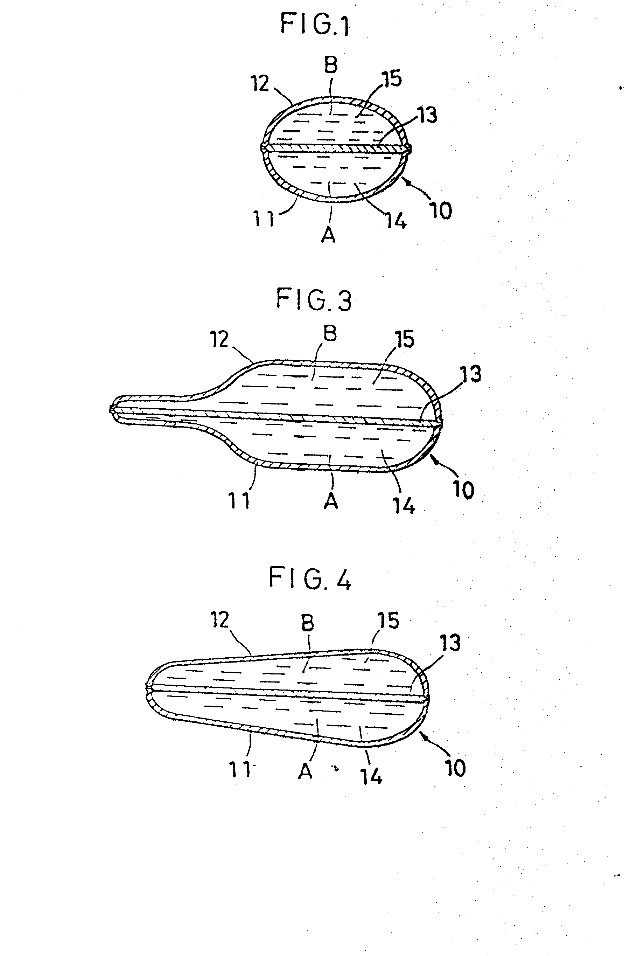

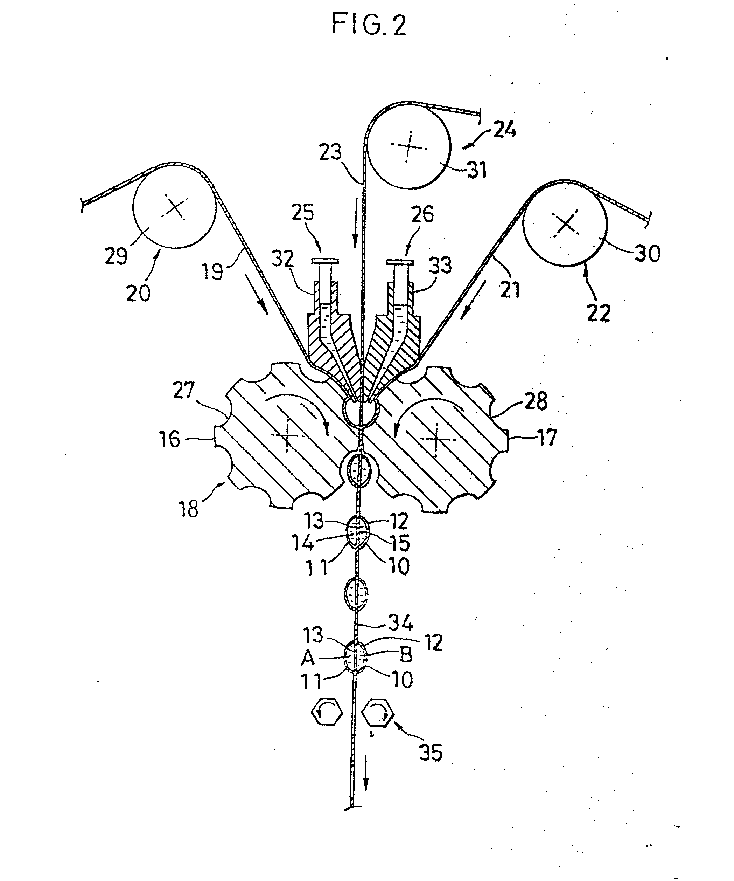

Fig. 1 shows an oval soft capsule 10. The capsule 10 comprises an approximately semispherical first shell 11 at one side, an approximately semispherical second shell 12 at the other side and a partition 13 provided therebetween. The three members 11, 12, 13 are joined together at their peripheries by compression. The two shells 11, 12 define therebetween a space, which is divided into two cells 14, 15 by the partition 13. The first cell 14 between the partition 13 and the first shell 11 contains first contents A (e.g. liquid medicinal), while the second cell 15 between the partition 13 and the second shell 12 contains second contents B (e.g. liquid medicinal).

Fig. 2 shows an example of apparatus for producing the soft capsule 10.

The production apparatus comprises a capsule former 18 composed of a pair of die rolls 16, 17, a first film feeder 20 for continuously feeding between the die rolls 16, 17 a first film 19 for forming the first shell 11, a second film feeder 22 for continuously feeding between the die rolls 16, 17 a second film 21 for forming the second shell 12, a third film feeder 24 for continuously feeding. a third film 23 for forming the partition 13 from between the first film 19 and the second film 21 to between the die rolls 16, 17, a first contents feeder 25 for placing the contents A into a space between the third film 23 and the first film 19 at capsule forming portions thereof, and a second contents feeder 26 for placing the contents B into a space between the third film 23 and the second film 21 at capsule forming portions thereof.

The die rolls 16, 17 are the same as those used for the conventional rotary apparatus for producing soft capsules. The outer peripheral surface of the first die roll 16 is formed with a multiplicity of generally semispherical film suction cavities 27 for shaping specified portions of the first film 19 to the form of the first shell 11 by vacuum suction. The cavities 27 are arranged at a predetermined spacing axially of the die roll 16 as well as circumferentially thereof. Although not shown, these cavities 27 communicate with a vacuum pump via a channel in the die roll 16. The outer peripheral surface of the second die roll 17 is also similarly formed with film suction cavities 28 corresponding to the cavities 27. The mating die rolls 16, 17 are arranged side by side horizontally and are rotated in the directions of arrows shown in Fig. 2 at the same speed so that the corresponding cavities 27, 28 are opposed to each other at the nip of the rolls and move downward at the same speed.

Although not shown, the first film feeder 20 is so adapted that a solution (e.g. gelatin solution) serving as the material for the first film 19 is supplied from a tank to a preheated rotating film forming roller to continuously form the first film 19 in the form of a strip. The film is passed over a plurality of unillustrated rollers, led to a guide roller 29 positioned close to the first die roll 16 thereabove, and fed to the nip of the die rolls 16, 17 obliquely from thereabove. The second film feeder 22 also has the same construction as above; the second film 21 is passed over a guide roller 30 close to and above the second die roll 17 and obliquely fed to the nip of the die rolls 16, 17 from thereabove. The third film feeder 24 also has the same construction as above. The third film 23 is passed over a guide roller 31 disposed above the nip of the die rolls 16, 17 and fed between the die rolls 16, 17 from immediately thereabove.

The first contents feeder 25 comprises a multiplicity of pumps 32 which are arranged immediately above the nip of the die rolls 16, 17, between the first film 19 and the third film 23 at the first die roll (16) side. These pumps 32 are arranged axially of the die roll 16 in corresponding relation to the cavities 27 of the die roll 16.

The casings of these pumps 32 also serve to guide and heat the films 19, 23. The second contents feeder 26, which has the same construction as bove, comprises a multiplicity of pumps 33 arranged immediately above the nip of the die rolls 16, 17, between the second film 21 and the third film 23 at the second die roll (17) side.

Of the three films 19, 21, 23 to be fed to the nip of the pair of die rolls 16, 17 in the above production apparatus, the first and second films 19, 21 are drawn into the cavities 27, 28 and shaped to the form of the first and second shells 11, 12 by vacuum suction before reaching the nip of the rolls 16, 17, whereby spaces are formed to provide interior cells 14, 15. While passing through the nip of the die rolls 16, 17, the three films 19, 21, 23 are compressed together progressively from below upward except at the capsule forming portions corresponding to the cavities 27, 28. When the films have been compressed together below and around the capsule forming portions, specified quantities of contents A, B are placed into each space between the third film 23 and the first film 19 now shaped in the form of the first shell 11 and into each space oetween the third film 23 and the second film 21 in the form of the second shell 12, respectively by the corresponding pumps 32, 33. The three films are thereafter compressed together above and around the capsule forming portions to completely shape capsules 10. The three films 19, 21, 23 delivered downward from the nip of the die rolls 16, 17 are in the form of a compressed sheet 34 except at the portions of capsules 10. While the sheet further moves downward, the soft capsules 10 are blanked out by the same device 35 as heretofore used. The capsules 10 are cleaned and dried in the same manner as conventionally practiced, whereby multicellular soft capsules 10 like the one shown in Fig. 1 are produced.

With the production apparatus described above, the pumps 32, 33 are used for injecting the contents A, B into the spaces, so that the encapsulation process involves only greatly reduced variations in the quantity of injection. With no contents injected into the compressed portion of the films 19, 21, 23, the loss of contents can be minimized to achieve a very high yield. Furthermore, highly viscous contents can be encapsulated similarly.

However, the multicellular soft capsule of the present invention can be prepared also by the conventional planar process. In this case, a first film for forming the first shell 11 is placed on a lower die, and a predetermined amount of first contents A are poured onto the film. Next, a third film for forming the partition 13 is placed over the contents, a specified amount of second contents B are poured onto the third film, and a second film for forming the second shell 12 is placed over the contents B. An upper die is placed over the second film, and pressure is applied to the die to compress the three films together except at the capsule forming portions thereof. Finally, the resulting capsule 10 is blanked out from the sheet by suitable means, followed by the same treatment as above.

Any of the materials usually used for the shell of soft capsules is usable for forming the shells and partition of the multicellular soft capsule of the present invention. An example of useful material is one consisting chiefly of gelatin and further containing a plasticizer, perfume, pigment, solubility adjusting agent, etc. added thereto as desired.

The medicinals and like contents to be enclosed in the multicellular soft capsule of the present invention are preferably liquid. When a solid is to be enclosed, it is preferably encapsulated in the form of a solution or suspension.

The multicellular soft capsule of the present invention is not limited to the oval shape described but may be of any shape, such as oblong form, round form, tubular form as seen in Fig. 3, or in the form of a suppository as shown in Fig. 4. Throughout Figs. 1, 3 and 4, like parts are referred to by like numerals.

The multicellular soft capsule of the present invention is usable not only for medicinals but also for cosmetics, food, etc. For cosmetics, tubular capsules are especially useful.

One portion of the multicellular soft capsule of the present invention can be made different from the other portion thereof in properties by suitably selectively using the rapidly soluble film, enteric film, prolonged release film, etc. exemplified below for the shells and partition of the capsule and by placing suitably selected contents into the two cells. In this case, the soft capsule can be given a beautiful appearance by making the first shell and the second shell different in color. The combination of such colors, when associated with the contents, is useful also for the quality control of the product to be obtained.

A rapidly soluble film is formed in the aforementioned manner, for example, from a gelatin solution prepared by heating 400 g of gelatin, 100 g of glycerin and 500 g of distilled water at about $60^{\circ}$ C with stirring.

An enteric film is prepared, for example, by dissolving 30 g of hydroxypropylmethylcellulose phthalate in 30 ml of 1N aqueous solution of sodium hydroxide, adding the solution to the mixture of 220 g of gelatin, 50 g of glycerin and 270 ml of distilled water, heating the mixture and treating the resulting gelatin solution in the same manner as above.

A prolonged release film is prepared, for example, from 250 g of gelatin, 30 g of agar powder,

50 g of glycerin and 300 ml of distilled water, by treating these materials in the same manner as in the case of the rapidly soluble film.

Specific examples of multicellular soft capsules of the present invention will be described below. The formulations in these examples show the quantities of compounds to be enclosed in one capsule.

Example 1

A 50 g quantity of 2-nitroxymethyl-6-chloro-pyridine is dissolved in 250 g of polyethylene glycol 400 and 400 g of glycerin to prepare contents A. Separately from this, 12 g of ethylcellulose is dissolved in 600 g of Miglyol (registered trademark) 812, and 50 g of 2-nitroxymethyl-6-chloropyridine is added to the solution to prepare contents B.

Using a rapidly soluble film (0.8 mm in thickness) prepared by the foregoing method as the first, second and third films 19, 21, 23 and further using the foregoing production apparatus which is equipped with No. 5 oval dies as the pair of die rolls 16, 17, the contents A, B are uniformly injected into first cells 14 and second cells 15 at the same time, in an amount of 0.12 ml in each cell, by the contents feeders 25, 26, respectively.

The die rolls 16, 17 are rotated at a film compression temperature of 40 to 50° C to produce 260 multicellular soft capsules per minute, each capsule weighing 485 mg.

| | | |
|---|---|---|
| A: | 2-Nitroxymethyl-6-chloropyridine | 10 mg |
| | Polyethylene glycol 400 | 50 mg |
| | Glycerin | 80 mg |
| B: | 2-Nitroxymethyl-6-chloropyridine | 10 mg |
| | Miglyol 812 | 120 mg |
| | Ethylcellulose | 2.4 mg |

Example 2

Multicellular soft capsules are obtained in the same manner as in Example 1 except that the following contents A, B are placed into the first and second cells 14, 15, respectively.

| | | |
|---|---|---|
| A: | Thioctic acid amide | 5 mg |
| | Riboflavin | 4 mg |
| | Vegetable oil | 100 mg |
| B: | Thiamine nitrate | 10 mg |
| | Tocopheryl acetate | 50 mg |
| | Vegetable oil | 50 mg |

Example 3

Multicellular soft capsules are obtained in the same manner as in Example 1 except that the following contents A, B are placed into the first and second cells 14, 15, respectively.

| | | |
|---|---|---|
| A: | Nitroglycerin | 2.5 mg |
| | Propylene glycol | 100 mg |

B: Nitroglycerin            2.5 mg

   Sudan Blue            0.2 mg

   Ethylcellulose          2 mg

   Panacet 800           100 mg

Example 4

Sustained release soft capsules are prepared, using the above-mentioned rapidly soluble film for the first shell 11 and the aforementioned enteric film for the partition 13 and for the second shell 12 and placing the following same contents into the two cells 14, 15.

A: 6-(3,4-Dimethoxyphenyl)-1-ethyl-4-mesitylimino-3-methyl-3,4-dihydro-2(1H)-pyrimidinone    10 mg

   Vegetable oil          100 mg

B: 6-(3,4-Dimethoxyphenyl)-1-ethyl-4-mesitylimino-3-methyl-3,4-dihydro-2(1H)-pyrimidinone    10 mg

   Vegetable oil          100 mg

Example 5

Sustained release soft capsules are prepared in the same manner as in Example 4 with the exception of using the aforementioned prolonged release film for the partition 13 and the second shell 12.

A: Nitroglycerin            3 mg

   Vegetable oil          100 mg

B: Nitroglycerin            3 mg

   Vegetable oil          100 mg

Example 6

Sustained release multicellular soft capsules in suppository type are prepared in the same manner as in Example 1 except that the following contents A, B are placed into the first and second cells 14, 15, respectively.

| | |
|---|---|
| A: Diclofenac sodium | 15 mg |
| Miglyol 812 | 150 mg |
| B: Diclofenac sodium | 35 mg |
| Metolose (registered trademark) 90SH 100 | 50 mg |
| Carbopol (registered trademark) 940 | 10 mg |
| Miglyol 812 | 100 mg |

Example 7

Reinforcement multicellular soft capsules are prepared in the same manner as in Example 1 except that the following contents A, B are placed into the first and second cells 14, 15 respectively.

| | |
|---|---|
| A: Vitamin A oil | 100,000 IU |
| L-ascorbic acid | 50 mg |
| Pyridoxine phosphate | 10 mg |
| Miglyol 812 | 40 mg |
| B: L-lysine hydrochloride | 5 mg |
| L-valine | 5 mg |
| L-leucine | 5 mg |
| L-threonine | 5 mg |
| L-tryptophan | 5 mg |

| | |
|---|---|
| L-methionine | 5 mg |
| L-phenylalanine | 5 mg |
| Miglyol 812 | 130 mg |

Example 8

Cream-containing tubular multicellular soft capsules are prepared in the same manner as in Example 1 except that the following contents A, B are placed into the first and second cells 14, 15, respectively.

| | |
|---|---|
| A: Vitamin A oil | 200,000 IU |
| Tocopheryl acetate | 10 mg |
| Monostearic acid glyceride | 2 mg |
| B: L-ascorbic acid | 10 mg |
| Polysorbate 80 | 2 mg |
| Propylene glycol | 150 mg |

Before use, the tip of the capsule is cut off, and the contents A, B are mixed together on the palm and then applied to the skin.

CLAIMS

1. A multicellular soft capsule having an interior space defined by a shell and divided into a plurality of cells by at least one partition.

2. A multicellular soft capsule as defined in claim 1 wherein the shell comprises a first shell portion at one side and a second shell portion at the other side, and said at least one partition is provided between the shell portions.

3. A multicellular soft capsule as defined in claim 1 wherein the cells are two in number.

4. A multicellular soft capsule as defined in claim 3 wherein the shell comprises a first shell portion at one side and a second shell portion at the other side, and one partition is provided between the shell portions.

5. A multicellular soft capsule as defined in claim 4 wherein the first shell portion, the second shell portion and the partition are each a rapidly or intragastrically soluble film or a prolonged release or enteric film.

6. A multicellular soft capsule as defined in claim 4 wherein the first shell portion is a rapidly or intragastrically soluble film, and the second shell portion and the partition are a prolonged release or enteric film.

7. A multicellular soft capsule as defined in claim 3 or 4 wherein a rapidly soluble medicinal is enclosed in one of the cells, and a prolonged release medicinal is enclosed in the other cell.

8. A process for producing multicellular soft capsules which comprise a first shell and a second shell defining a space therebetween and at least one partition provided between the shells and dividing the space into a plurality of cells, the process comprising feeding to capsule forming means a first film for forming the first shell, a second film for forming the second shell and at least one sheet of third film for forming the partition with the third film positioned between the first and second films, and compressing the first, second and third films together except at capsule forming portions thereof so that contents are placed into spaces between the films at the capsule forming portions.

9. A process for producing multicellular soft capsules which comprise a first shell and a second shell defining a space therebetween and one partition provided between the shells and dividing the space into two cells, the process comprising feeding to capsule forming means a first film for forming the first shell, a second film for forming the second shell and one sheet of third film for forming one partition with the third film positioned between the first and second films, and compressing the three films together except at capsule forming portions thereof so that contents are placed into a space between the third film and the first film and into a space between the third film and the second film at the capsule forming portions.

10. A process for producing multicellular soft capsules as defined in claim 9 wherein the capsule forming means is a pair of die rolls, and the three films are continuously fed between the die rolls, the three films being compressed together except at the capsule forming portions thereof while placing the contents into the space between the third film and the first film and into the space between the third film and the second film at the capsule forming portions.

11. An apparatus for producing multicellular soft capsules which comprise a first shell and a second shell defining a space therebetween and at least one partition provided between the shells and dividing the space into a plurality of cells, the apparatus comprising capsule forming means composed of a pair of die rolls, first film feeding means for continuously feeding between the die rolls a first film for forming the first shell, second film feeding means for continuously feeding between the die rolls a second film for forming the second shell, third film feeding means for continuously feeding at least one sheet of third film for forming the partition from between the first film and the second film to between the die rolls, and a plurality of contents feeding means for separately placing contents into spaces between the films at capsule forming portions thereof.

12. An apparatus for producing multicellular soft capsules which comprise a first shell and a second shell defining a space therebetween and one partition provided between the shells and dividing the space into two cells, the apparatus comprising capsule forming means composed of a pair of die rolls, first film feeding means for continuously feeding between the die rolls a first film for forming the first shell, second film feeding means for continuously feeding between the die rolls a second film for forming the second shell, third film feeding means for continuously feeding one sheet of third film for forming one partition from between the first film and the second film to between the die rolls, first contents feeding means for placing contents into a space between the third film and the first film at capsule forming portions thereof, and second contents feeding means for placing contents into a space between the third film and the second film at capsule forming portions thereof.

13. An apparatus for producing multicellular soft capsules as defined in claim 12 wherein the pair of die rolls are arranged side by side horizontally, and the third film feeding means feeds its film to between the die rolls from immediately thereabove, the first and second film feeding means being adapted to feed their films to between the die

22

rolls obliquely from thereabove, the contents feeding means being adapted to place the contents into the spaces between the films from thereabove at the capsule forming portions.

14. An apparatus for producing multicellular soft capsules as defined in claim 13 wherein each of the contents feeding means comprises pumps arranged between the films concerned immediately above the die roll.

0211079

## FIG.1

## FIG.3

## FIG.4

# FIG.2

0211079

# INTERNATIONAL SEARCH REPORT

International Application No. PCT/JP85/00041

## I. CLASSIFICATION OF SUBJECT MATTER (If several classification symbols apply, indicate all) 9

According to International Patent Classification (IPC) or to both National Classification and IPC

Int.Cl$^4$ A61K 7/00, A61K 9/48, A61J 3/07, A23P 1/04

## II. FIELDS SEARCHED

| Minimum Documentation Searched 4 | |
|---|---|
| Classification System | Classification Symbols |
| IPC | A61K 7/00, A61K 9/48, A61J 3/07, A61J 1/00, A61K 9/00, B65B 9/04 |

Documentation Searched other than Minimum Documentation
to the Extent that such Documents are Included in the Fields Searched 5

| | |
|---|---|
| Jitsuyo Shinan Koho | 1926 – 1985 |
| Kokai Jitsuyo Shinan Koho | 1971 – 1985 |

## III. DOCUMENTS CONSIDERED TO BE RELEVANT 14

| Category* | Citation of Document, 16 with indication, where appropriate, of the relevant passages 17 | Relevant to Claim No. 18 |
|---|---|---|
| X | DE, A, 2317140 (Wella AG) 17 October 1974 (17. 10. 74) Column 9, lines 2 to 10 (Family nashi) | 1 – 9 |
| X | DE, A, 2052668 (Gillette Co.) 6 May 1971 (06. 05. 71) Column 11, lines 2 to 26 & SE, B, 355492 & CA, A1, 942673 & AT, B, 316757 | 1 – 9 |
| X | JP, A, 55-122710 (Kyoto Ceramic Kabushiki Kaisha) 20 September 1980 (20. 09. 80) Column 4, line 12 to column 5, line 9 & DE, A1, 3005350 & GB, B2, 2043444 & US, A, 4293540 | 1 – 9 |
| X | JP, B1, 45-359 (Jacob A. Glassman) 8 January 1970 (08. 01. 70) Column 4, lines 32 to 40, (Family nashi) | 1 – 9 |
| X | JP, Y1, 16-1127 (Takeuchi Kintaro) 1 February 1941 (01. 02. 41) Column 1 (Family nashi) | 1 – 9 |
| X | JP, Y1, 16-11981 (Takeuchi Kintaro) 16 August 1941 (16. 08. 41) Column 1 (Family nashi) | 1 – 9 |

* Special categories of cited documents: 15

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search 2 | Date of Mailing of this International Search Report 2 |
|---|---|
| April 6, 1985 (06. 04. 85) | April 22, 1985 (22. 04. 85) |
| International Searching Authority 1 | Signature of Authorized Officer 10 |
| Japanese Patent Office | |

Form PCT/ISA/210 (second sheet) (October 1981)

| FURTHER INFORMATION CONTINUED FROM THE SECOND SHEET | | |
|---|---|---|
| X | JP, A, 59-22552 (Fuji Capsule Kabushiki Kaisha) 4 February 1984 (04. 02. 84) Column 5, Fig. 1 (Family nashi) | 10 - 14 |
| X | JP, A, 57-86351 (Tokai Capsule Kabushiki Kaisha) 29 May 1982 (29. 05. 82) Column 3, Fig. 1 (Family nashi) | 10 - 14 |

**V.☐ OBSERVATIONS WHERE CERTAIN CLAIMS WERE FOUND UNSEARCHABLE [10]**

This international search report has not been established in respect of certain claims under Article 17(2) (a) for the following reasons:

1.☐ Claim numbers_____ because they relate to subject matter [12] not required to be searched by this Authority, namely:

2.☐ Claim numbers_____ because they relate to parts of the international application that do not comply with the prescribed requirements to such an extent that no meaningful international search can be carried out [13], specifically:

**VI.☐ OBSERVATIONS WHERE UNITY OF INVENTION IS LACKING [11]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

**Remark on Protest**

☐ The additional search fees were accompanied by applicant's protest.

☐ No protest accompanied the payment of additional search fees.